# DEMANDE DE BREVET EUROPEEN

(11) **EP 1 900 810 A1**
(43) Date de publication de la demande: **19.03.2008**
(21) Numéro de dépôt: 06291462.7
(22) Date de dépôt: 15.09.2006
(51) Int. Cl.: C12N 5/06

(54) **Methode d'extraction et de selection de cellules**

(71) Demandeur: Celogos, 75724 Paris Cedex 15 (FR)
(72) Inventeur: Pinset, Christian, 75018 Paris (FR)
(74) Mandataire: Hirsch & Associés

(57) **Abrégé**

Procédé de sélection de cellules comprenant au moins une étape au moins partiellement combinée de digestion enzymatique et de sélection en culture.

## Description

### DOMAINE DE L'INVENTION

La présente invention se rapporte à des procédés d'extraction, de sélection, de conservation de cellules pour la production de cellules pouvant être utilisées pour la thérapie cellulaire et la pharmacologie ainsi que des milieux de culture spécifiquement adaptés.

### ART ANTERIEUR

La publication Cosimo de Bari et al (Arthritis and Rheumatism, vol 44, n°8, August 2001, p 1928 à 1942) décrit l'obtention de cellules synoviales par la digestion de membrane synoviale avec de la collagenase pendant 24 heures en culture.

Le document WO2004/055174 décrit un procédé de sélection puis d'amplification de myoblastes. L'étape de sélection est postérieure à l'étape d'extraction des cellules musculaires par digestion enzymatique.

### RESUME DE L'INVENTION

L'invention se rapporte à un procédé de sélection de cellules, de préférence musculaires, combinant dans la même étape ou au moins partiellement, l'extraction enzymatique et la sélection. L'invention se rapporte aussi aux cellules ainsi obtenues et à leur utilisation thérapeutique. L'invention se rapporte également à un procédé de sélection de cellules, de préférence musculaires, par la congélation dans un milieu contenant de la Tréhalose. L'invention se rapporte aussi à des milieux de culture spécifiquement adaptés à la mise en oeuvre de ces procédés.

Plus spécifiquement l'invention se rapporte à :
1. Un procédé de sélection de cellules comprenant au moins une étape au moins partiellement combinée de digestion enzymatique et de sélection en culture.
2. Un procédé selon le point 1 dans lequel la source des cellules est un échantillon tissulaire et de préférence une biopsie musculaire.
3. Un procédé selon le point 1 dans lequel la source des cellules est une culture de cellules et de préférence un agrégat de cellules en culture.
4. Un procédé selon les points 1 à 3 dans lequel les cellules sélectionnées sont des cellules musculaires.
5. Un procédé selon les points 1 à 4 dans lequel les cellules sélectionnées sont des cellules souches du muscle squelettique, des cellules souches musculaires, des cellules souches musculaires précurseurs, des myoblastes ou des cellules satellites.
6. Un procédé selon les points 1 à 5 dans lequel la sélection se fait au moins par la culture dans un milieu de sélection.
7. Un procédé selon les points 1 à 5 dans lequel la sélection se fait au moins par l'adhésion à un substrat en culture.
8. Un procédé selon les points 1 à 5 dans lequel la sélection se fait au moins par l'adhésion à un substrat et par culture dans un milieu de sélection.
9. Un procédé selon les points 7 à 8 dans lesquelles le substrat est choisi dans le groupe consistant en le verre, le plastique traité, des substrats synthétiques, des cellules nourricières, des anticorps, des peptides et les constituants de la matrice extra cellulaire, de préférence la Laminine, la Fibronectine, la Vitronectine.
10. Un procédé selon le point 9 dans lequel les cellules adhérentes sont sélectionnées.
11. Un procédé selon le point 9 dans lequel les cellules non adhérentes sont sélectionnées.
12. Un procédé selon l'un des points précédents dans lesquelles la sélection se fait par culture dans un milieu de sélection comprenant au moins de la dexaméthasone, du sélénium, et un ou plusieurs composés choisi dans le groupe consistant en l'acide ascorbique-2-phosphate, l'acide ascorbique et leurs mélanges.
13. Un procédé selon l'un des points précédents dans lequel le milieu de culture comprend l'enzyme utilisée pour la digestion enzymatique.
14. Un procédé selon l'un des points précédents dans lequel le milieu de sélection comprend l'enzyme utilisée pour la digestion enzymatique.
15. Un procédé selon l'un des points précédents dans lequel l'extraction enzymatique utilise une enzyme sélectionnée dans le groupe consistant en la collagènase, la protéase neutre, la pronase, la trypsine et leurs mélanges.
16. Un procédé selon l'un des points précédents dans lequel l'étape combinée d'extraction enzymatique et de sélection dure au moins 3 heures, de préférence au moins 6 heures, de manière préférée au moins 12 heures et de manière très préféré au moins 24 heures, et de manière préférée entre toutes au moins 48 heures.
17. Un procédé de sélection de cellules musculaires par conservation en présence d'un composé choisi dans le groupe consistant en les sucres, la tréhalose, la glycine, le HES (hydroxyethyl starch), le glycerol et l'arbutin.
18. Un procédé selon le point 17 dans lequel la source des cellules est un échantillon tissulaire et de préférence une biopsie musculaire.
19. Un procédé selon le point 17 dans lequel la source des cellules est une culture de cellules et de préférence un agrégat de cellules en culture.
20. Un procédé selon les points 17 à 19 dans lequel les cellules sélectionnées sont des cellules souches du muscle squelettique, des cellules souches musculaires, des cellules souches musculaires précurseurs, des myoblastes ou des cellules satellites.
21. Un procédé selon les points 17 à 20 dans lequel la conservation comprend au moins une étape de congélation.
22. Un procédé selon le point précédent dans laquelle la congélation se fait en présence de tréhalose.
23. Un procédé selon l'un des points 21 ou 22 dans laquelle la concentration de tréhalose est comprise entre 1mM à 1M de préférence 0.2M.
24. Cellules susceptibles d'être obtenues par les procédés de sélection de cellules des points précédents.
25. Cellules musculaires susceptibles d'être obtenues par les procédés de sélection de cellules des points précédents.
26. Cellules musculaires susceptibles d'être obtenues par les procédés de sélection de cellules selon l'un des points précédents pour leur utilisation en thérapie cellulaire.
27. Cellules musculaires selon le point 26 pour leur utilisation dans le traitement fonctionnel des muscles.
28. Cellules musculaires selon le point précédente pour leur utilisation dans le traitement fonctionnel des petits muscles.
29. Cellules musculaires selon le point précédente pour leur utilisation dans le traitement fonctionnel des sphincters.
30. Cellules musculaires selon le point précédente pour leur utilisation dans le traitement fonctionnel de l'incontinence urinaire
31. Cellules musculaires selon le point 29 pour leur utilisation dans le traitement fonctionnel de l'incontinence anale.

Les Figures sont décrites ci-dessous:

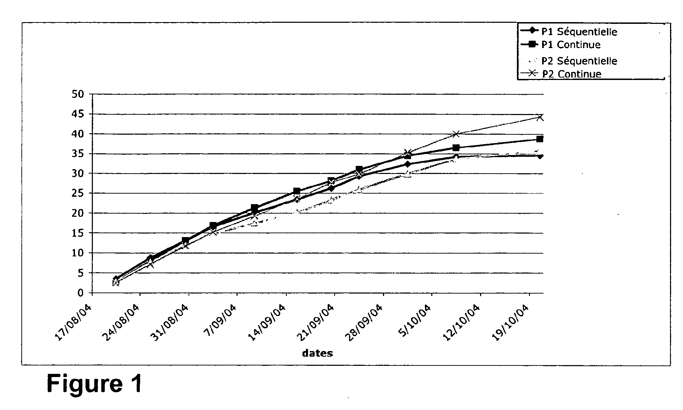

Figure 1: cette figure montre le nombre de division cumulée en fonction du temps pour des cellules issues du patient 1 et sélectionnées par une extraction séquentielle (représenté par des losanges sur le graphique), ou sélectionnées par une extraction continue (représenté par des carrés sur le graphique) ainsi que des cellules issues du patient 2 et sélectionnées par une extraction séquentielle (représenté par des triangles sur le graphique), ou sélectionnées par une extraction continue (représenté par des croix sur le graphique).

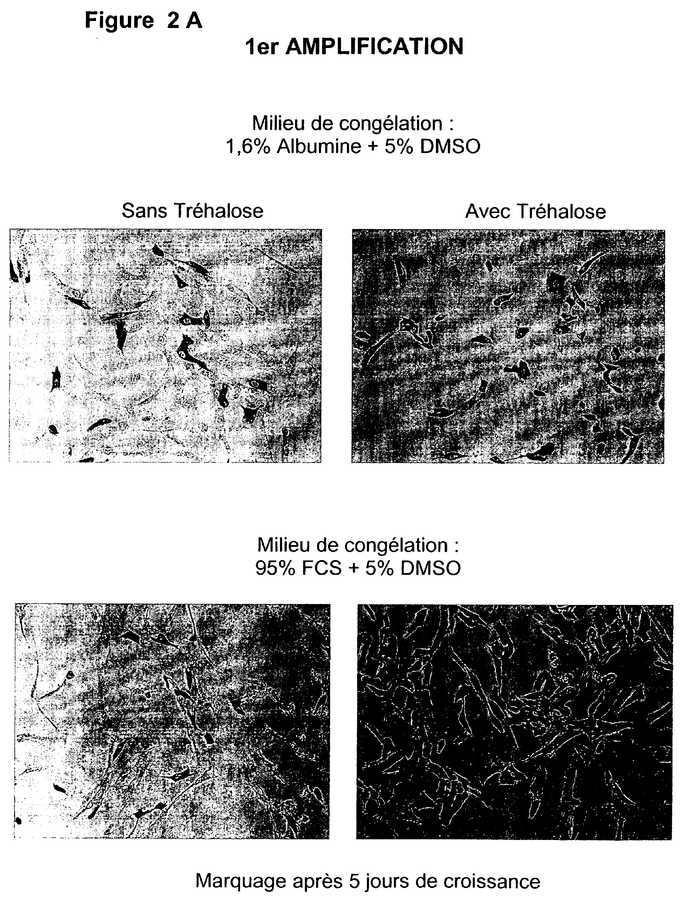

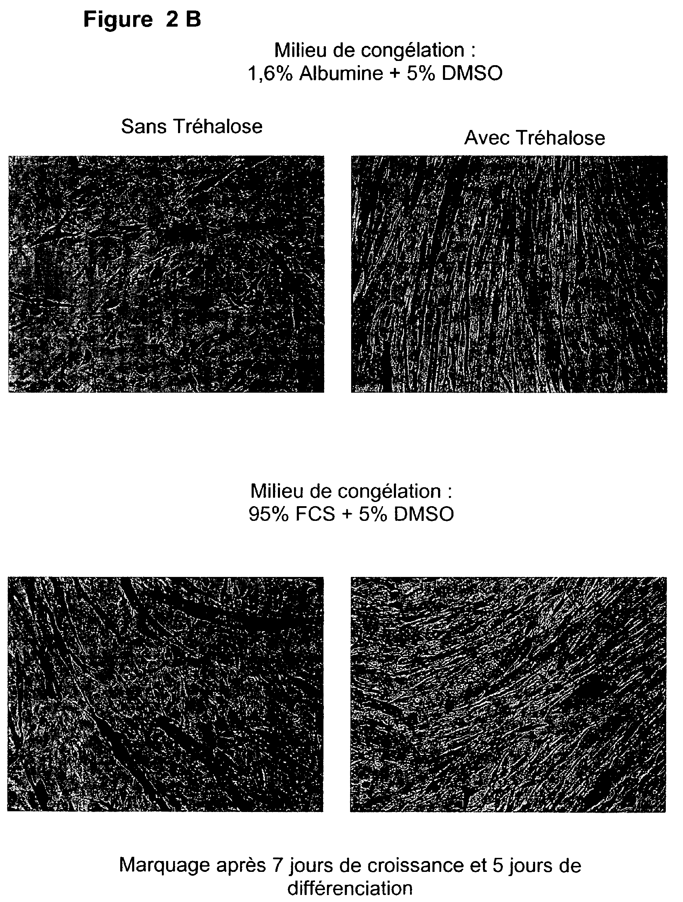

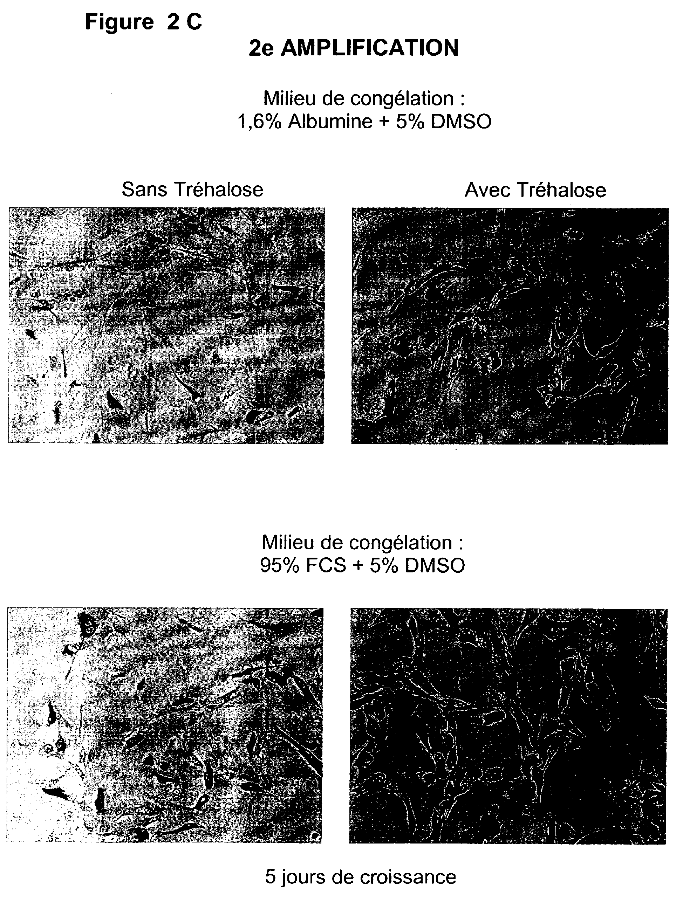

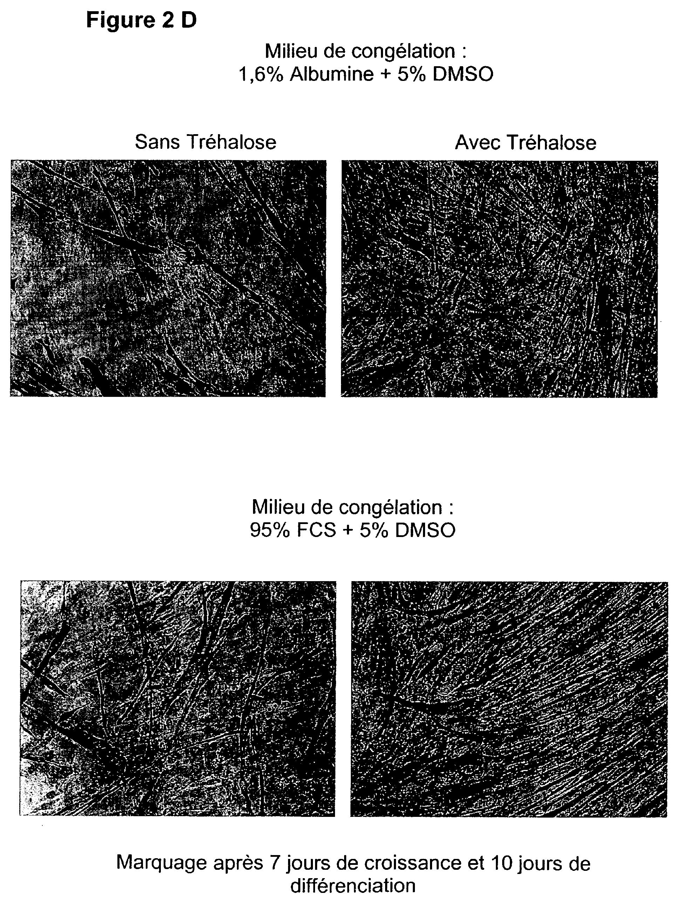

Figure 2 Cette figure montre des cellules issues des procédés d'extraction et de sélection sur du tissu musculaire ayant été préalablement conservées par congélation en absence ou en présence de tréhalose Les cellules musculaires apparaissent teintées en brun.
   La figure 2A compare le marquage pour la desmine lors d'une première amplification après 5 jours de croissance pour deux milieux, l'un avec 1,6% Albumine + 5% DMSO (figures du haut) et l'autre avec 95% FCS + 5% DMSO (figures du bas), tout les deux avec ou sans ajout de tréhalose à une concentration finale de 0.2M (la colonne de gauche représente les résultats sans tréhalose et la colonne de droite, ceux avec tréhalose). La figure 2B compare le marquage pour la desmine lors d'une première amplification après 7 jours de croissance et 5 jours de différenciation pour deux milieux, l'un avec 1,6% Albumine + 5% DMSO (figures du haut) et l'autre avec 95% FCS + 5% DMSO (figures du bas), tout les deux avec ou sans ajout de tréhalose à une concentration finale 0.2M (la colonne de gauche représente les résultats sans tréhalose et la colonne de droite, ceux avec tréhalose). La figure 2C compare le marquage pour la desmine lors d'une deuxième amplification après 5 jours de croissance pour deux milieux, l'un avec 1,6% Albumine + 5% DMSO (figures du haut) et l'autre avec 95% FCS + 5% DMSO (figures du bas), tout les deux avec ou sans ajout de tréhalose à une concentration finale de 0.2M (la colonne de gauche représente les résultats sans tréhalose et la colonne de droite, ceux avec tréhalose). La figure 2D compare le marquage pour la desmine lors d'une deuxième amplification après 7 jours de croissance et 10 jours de différentiation pour deux milieux, l'un avec 1,6% Albumine + 5% DMSO (figures du haut) et l'autre avec 95% FCS + 5% DMSO (figures du bas), tout les deux avec ou sans ajout de tréhalose à une concentration finale de 0.2M (la colonne de gauche représente les résultats sans tréhalose et la colonne de droite, ceux avec tréhalose).

### DESCRIPTION DE L'INVENTION

La présente invention se rapporte à un procédé de production, de préservation de cellules, de préférence musculaires à partir de tissus biologiques ou d'agrégats cellulaires

Un des objectifs de cette nouvelle méthode est de simplifier et d'augmenter l'efficacité des différentes procédures nécessaires à l'obtention et la conservation de cellules pour la thérapie cellulaire et la pharmacologie et pour la constitution de banque tissulaire biologique à partir de biopsie tissulaire. Ces simplifications des procédures de production facilitent l'automatisation des procédés de culture et sécurisent les procédés de production.

Dans les procédés d'extraction cellulaire et de sélection de l'art anterieur, il était nécessaire d'avoir d'abord une étape d'extraction enzymatique, puis après un lavage et une neutralisation des enzymes, une étape séparée sélection des cellules. L'extraction enzymatique était de courte durée et séparée des étapes d'ensemencement et de sélection.

Il a été découvert qu'il était possible de combiner, au moins partiellement, l'étape d'extraction de cellules à partir de tissus (ou d'agrégats cellulaires) et l'étape de sélection cellulaire par culture. De plus cette combinaison résulte de manière particulièrement surprenante en une amélioration quantitative (rendement d'extraction) et qualitative (potentiel de croissance et de différenciation). Cette méthode est plus simple, plus rapide à mettre en oeuvre et plus efficace pour la production de cellules à usage thérapeutiques.

L'étape de sélection peut se faire sur différents critères tels que la capacité à répondre à des facteurs de croissance spécifiques qui résultent en une survie cellulaire, une croissance cellulaire différente ou encore un développement phénotypique différent. Un autre critère est la capacité d'adhésion sélective. Ces critères peuvent être combinés par la culture dans un milieu sélectionné et sur un substrat permettant l'adhésion sélective.

Ce procédé d'extraction combiné à l'étape de sélection continue permet notamment:
○ D'effectuer l'extraction par digestion enzymatique dans des conditions permettant de maintenir la viabilité et les propriétés biologiques des cellules.
○ De combiner en une seule étape l'extraction et la sélection cellulaire.
○ De pouvoir effectuer ce procédé d'extraction sélection sur des tissus directement congelés ;
○ De diminuer le nombre de manipulations humaines.
○ De diminuer la variabilité du procédé de production.
○ De diminuer les temps de culture pour la production du nombre de cellules nécessaires à des applications thérapeutiques.
○ De produire des cellules ayant les spécifications de cellules thérapeutiques pour la thérapie cellulaire à la fois pour la sécurité et l'efficacité.

La source des cellules peut être une biopsie ou aussi bien un agrégat cellulaire issu de culture de cellules primaire ou secondaire. Les procédés combinés de sélection et de digestion de l'invention peuvent également être utilisés sur toutes cellules susceptibles d'être extraites d'un groupe de cellules. L'invention trouve donc à s'appliquer aussi bien à des tissus issus d'une biopsie qu'à des culture cellulaires contenants notamment des cellules adhérentes ou des agrégat de cellules.

Les différentes étapes du procédé sont décrites ci-dessous.

### 1) Extraction et sélection cellulaire

Le principe de cette méthode d'extraction est d'initier l'extraction cellulaire enzymatique directement dans les milieux de culture permettant 1) le maintien des propriétés biologiques des cellules, dont la survie, 2) l'attachement différentiel et 3) la prolifération cellulaire.

Pour cet objectif, il faut sélectionner des enzymes dont l'action n'est pas inhibée par des facteurs de croissance, des composants sériques et qui n'altèrent pas le potentiel cellulaire. Un autre élément du choix est de pouvoir disposer d'enzymes qui ne présentent pas de danger sanitaire potentiel. Les milieux de culture sont composés de milieu de type DME/F12 aMEM, MCDB 120 et de facteurs de croissance ainsi que de composés sériques d'origine animale ou humaines. Les enzymes utilisées sont de type collagénase, libérase, protéase neutre, pronase seuls ou en combinaisons. Il est possible d'y adjoindre des agents chélateur de type EDTA ou EGTA.

### Les différents éléments du procédé d'extraction cellulaire sont:

### Dissociation mécanique.

Les fragments biopsiques sont émincés mécaniquement à l'aide de ciseaux chirurgicaux ou de scalpels à usage unique. Cette étape est optionnelle. Après cette étape de dissociation mécanique les fragments tissulaires peuvent être directement congelés dans les conditions adéquates. Les milieux de congélations sont soit des milieux définis soit des milieux contenant des sérums animaux ou humains. Comme agent cryo-préservant il est possible d'utiliser du glycérol, du DMSO, de la tréhalose, des sucres sous forme de monoosides ou de polyosides, de la glycine, la et du HES (hydroxyethyl starch). Cette conservation peut constituer une étape de sélection.

### Extraction enzymatique

Les fragments émincés sont repris dans les milieux de culture en présence d'enzymes et sont placés dans des flacons de culture. Ces flacons de culture sont incubés pendant des périodes pouvant aller jusqu'à plusieurs jours dans un incubateur à 37° en présence de gaz dont le CO₂ et l'Oxygène. La durée de digestion peut excéder 48 heures.

Cette étape permet de libérer les cellules du tissu dans des conditions maintenant leur viabilité, leur capacité d'adhésion préférentielle et leur capacité de répondre aux facteurs de croissance.

Cette méthode permet aussi de simplifier les procédures en limitant le nombre de manipulations techniques qui sont, dans ce procédé, réduites à mettre en présence les fragments tissulaires avec la solution enzymatique dans des conditions permettant la culture cellulaire. Cette étape est donc facilement automatisable. Les concentrations d'enzymes utilisées sont variables. Dans cette étape, les milieux de culture sont composés de milieux de type DME/F12 aMEM, MCDB 120 et de facteurs de croissance et de composés sériques d'origine animale ou humaines. Les choix des milieux de culture permettent de maintenir la viabilité cellulaire pendant la période d'extraction et d'initier la croissance et la sélection. A titre d'exemple, il est possible d'utiliser des milieux ce culture de type DME/F12 aMEM, MCDB 120 additionnés de protéines animales ou humaines. Pour cette étape il est aussi possible d'utiliser des milieux complètement synthétiques. L'origine de ces protéines peut être extractive ou synthétique. Parmi ces protéines, il est possible d'utiliser des protéines sériques (sérum, albumine ou transferrine) des facteurs de croissances (FGFs, HGF, IGFs, VEGF, EGF ou heréguline). Il est aussi possible d'additionner aux milieux de culture des vitamines (Vitamine C, dérivées de la vitamine A, vitamine B ou vitamine D), des hormones (stéroïdes , Insuline, hormones ou thyroïdiennes) et des agents pharmacologiques (Agonistes , antagonistes, inhibiteur ou activateur de voies métaboliques).

Ces procédés d'extraction peuvent être effectué sur des fragments tissulaires frais ou des fragments tissulaires congelés. Ce procédé permet d'allonger la période de traitement enzymatique sans altérer la viabilité cellulaire et en initiant les étapes de sélection.

### Sélection cellulaire

Les tissus cellulaires sont toujours composés de nombreux types cellulaires et un des objectifs de ce procédé est d'augmenter l'homogénéité et la fonctionnalité des cellules produites en utilisant ce procédé d'extraction et de sélection. Pour réaliser ces objectifs, il existe de nombreuses stratégies. Pendant l'étape de dissociation enzymatique, la sélection cellulaire peut être effectuée sur des propriétés biologiques (capacités de survie, cytoxicité sélective, expression génétique, capacité d'adhésion). De préférence dans ce procédé, la sélection se fait sur au moins deux propriétés biologiques.

### Définition des milieux de sélection

La survie cellulaire est dépendante à la fois de type cellulaire et de combinaisons d'éléments comme des sources de carbone (glucose, galactose fructose ou pyruvate), d'acides aminés, de sources de gaz (O₂ ou CO₂) de sources de facteurs de croissance, de sources de vitamines et des sources de purine et de pyrimidines.

La définition du type de milieux de sélection sera dépendante du type de cellules à obtenir. La capacité à métaboliser différentes sources de carbone, à répondre à des facteurs de croissance, à présenter une dépendance à certaines vitamines est variable en fonction des types cellulaires.

Les méthodes décrites sont particulièrement adaptées à la sélection des :
1) Cellules souches du muscle squelettique. Cette population cellulaire est dérivée d'une biopsie muscle squelettique qu possède un potentiel de réplication cellulaire et un potentiel de différenciation dans plusieurs lignages distincts dont au moins le muscle squelettique. A ce stade, cette population n'exprime pas la desmine.
2) Cellules souches musculaires précurseurs. Cette population cellulaire est dérivée d'une biopsie muscle squelettique qui possède un potentiel de réplication cellulaire et un potentiel de différenciation dans le lignage du muscle squelettique. A ce stade cette population n'exprime pas la desmine.
3) Myoblastes. Cette population cellulaire est dérivée d'une biopsie muscle squelettique qui possède un potentiel de réplication cellulaire et dont une proportion exprime la desmine.
   Ces trois types de cellules ainsi définies sont nommées les cellules musculaires. Les cellules musculaires produites par ces méthodes sont particulièrement utiles pour la thérapie cellulaire autologue ou allogénique
   Le document WO2004/055174 décrit des milieux de sélection adaptés à la production de myoblastes. L'invention décrit également des milieux de sélection améliorés. Ces milieux peuvent être dépourvu d'insuline et/ou contenir de l'acide ascorbique-2-Phosphate qui est un précurseur de l'acide ascorbique et permet d'augmenter la biodisponibilité et la stabilité in vitro de l'acide ascorbique pour la culture cellulaire.

### Utilisation de substrats pour la sélection par l'adhésion différentielle

Pendant l'étape de dissociation enzymatique, les fragments tissulaires peuvent être placés sur des supports de culture en présence de différents types de substrats. Par ce procédé, il est donc possible de séparer les cellules qui vont adhérer au substrat (cellules adhérentes) et les cellules qui ne vont pas adhérer au substrat (cellules non adhérentes).

Dans cette optique, il est possible d'utiliser différents types de substrats:
Des substrats synthétiques tels que du verre, des plastiques non traités (boite de bactériologie), des plastiques traitées avec des substrats synthétiques comme le polyomithine et Poly L lysine.
Des substrats biologiques tel que des protéines de la matrice extracellulaire (de la Laminine, de la Fibronectine, ou de la Vitronectine), de la matrice extracellulaire reconstituée, des "Feeder" de cellules nourricières ou des boites recouvertes d'anticorps spécifiques ou de peptides spécifiques.

Après un temps déterminé, il est possible de séparer deux types de cellules: les cellules non adhérentes et les cellules adhérentes

Les cellules non adhérentes sont récupérées par pipetage. Cette suspension cellulaire de cellules non adhérentes ainsi obtenue est une source de cellules pour la conservation cellulaire, l'amplification cellulaire et la thérapie cellulaire.

Les cellules adhérentes sont les cellules qui restent attachées aux substrats après pipetage. Les cellules sont alors soient amplifiées directement soit repiquées à l'aide solution de trypsine EDTA. Ces cellules sont également une source de cellules pour la conservation cellulaire, l'amplification cellulaire et la thérapie cellulaire.

### 2) Amplification cellulaire

Après la phase de sélection, il peut être utile de procéder à une phase d'amplification durant laquelle le nombre de cellules augmente par division cellulaire successive dans un milieu de croissance. Des milieux de culture adaptés à l'amplification cellulaire sont connus de l'homme du métier et incluent pour les myoblastes ceux divulgués dans la demande de brevet WO2004/055174 en plus de ceux divulgués par la présente invention.

### 3) Conservation cellulaire

Finalement une étape de congélation peut être mise en oeuvre. Celle-ci permet le stockage de longue durée des cellules produites en vue d'une utilisation ultérieure. Le document WO2004/055174 décrit des méthodes de congélation adaptée aux cellules musculaires. Les tissus et les cellules peuvent êtres congelées dans des milieux complètement exempts de protéines animales et en présence d'agents cryoprotecteurs comme le tréhalose qui permet la préservation sélective des cellules musculaires.

### 4) Caractérisation cellulaire fonctionnelle

Après l'étape de sélection, d'amplification ou avant utilisation des cellules, il peut être nécessaire de caractériser ces cellules. Nous définissons des caractérisations fonctionnelles et phénotypiques qui sont essentielles pour l'utilisation de cellules en tant que cellules thérapeutiques pour réparer un type cellulaire particulier.

### Test de croissance cellulaire

L'évaluation systématique du temps de doublement permet de caractériser la cinétique de croissance cellulaire de chaque échantillon produit.

Ce test permet de définir le taux de recouvrement cellulaire et le temps de doublement reflétant l'activité cellulaire intrinsèque. En cas de temps de doublement trop élevé (>35 heures), les cellules réinjectées ont potentiellement perdu leur capacité régénérative *in vivo.*

Une quantité définie de cellules est ensemencée dans des plaques multipuits. Après dépôt de milieu de croissance cellulaire, la quantification du contenu cellulaire obtenu entre 6h et 24h permet de définir le taux de recouvrement cellulaire. Des lectures successives à des intervalles de temps de culture définis permettent de mesurer le temps de doublement cellulaire.

Une fois fixées, les plaques peuvent être analysées par un dispositif automatisé ou visuellement par un opérateur.

Cette technologie peut être gérée par un logiciel programmé pour obtenir une quantification automatique des cellules à partir de l'analyse microscopique.

L'analyse automatique se fait en 3 étapes, étant l'acquisition des images, la transformation des images en chiffres grâce à un logiciel d'acquisition et la représentation éventuelle des chiffres sous forme de graphiques pour la validation grâce à un logiciel d'exploitation.

L'analyse d'image quantitative par un logiciel adapté au microscope optique inversé

Eclipse TE2000™ permet de définir le nombre de cellules obtenues, donc leur capacité de croissance. En effet, la programmation du logiciel permet de calculer à partir de la quantité cellulaire, le nombre de divisions obtenu et de déduire du nombre de divisions, le temps de doublement.

### Test clonogénique

Ce test permet de contrôler les échantillons en explorant la capacité des cellules à former des colonies (efficacité clonale) et à se différencier.

Le principe repose sur l'analyse de la croissance à basse densité dans un milieu de croissance (ou d'expansion) dans un premier temps et sur l'évaluation de la capacité à se différencier dans un milieu spécifique de différentiation dans un second temps. Les cellules sont ensemencées dans un milieu de croissance pendant 14 jours. La moitié des cellules est fixée et colorée au bout de 14 jours et l'autre moitié est fixée et colorée au bout de 7 jours de culture supplémentaire dans un milieu de différenciation.

On procède à : 1) une observation macroscopique qui permet de dénombrer le nombre total de colonies et de déterminer ainsi le pourcentage de cellules qui présente un potentiel de croissance clonale appelé efficacité clonale; et 2) une observation microscopique qui permet de déterminer le nombre et le pourcentage de colonies de cellules musculaires. Pour les cellules musculaires, les colonies de précurseurs musculaires forment par fusion cellulaire des cellules polynuclées fusiformes : les myotubes qui dans l'organisme formeront les fibres musculaires.

Une colonie est considérée comme composée de précurseurs musculaires lorsqu'elle contient au moins un myotube, sachant que le myotube contient au moins 3 noyaux. Ce pourcentage représente le taux de différenciation.

Au final, des prises d'essais contenant 50 cellules sont ensemencées dans des flacons de 25 cm², le nombre de colonie dénombré correspondant au pourcentage direct de cellules ayant une capacité clonale. On observe également le nombre de cellules s'étant différenciées en colonies myogéniques dans les flacons contenant du milieu de différenciation. Ce test permet donc de définir l'efficacité clonale et le taux de différenciation.

### Caractérisation phénotypique

Cette caractérisation repose sur la mise en évidence d'un marqueur phénotypique. A titre d'exemple, pour le muscle il est possible d'utiliser une protéine de structure musculaire, le filament intermédiaire de la desmine. Les myoblastes sont positifs pour la desmine. Le marquage spécifique de cette protéine musculaire à l'aide d'un anticorps monoclonal permet une identification et une mesure de la population cellulaire étudiée. Cette analyse consiste à marquer les cellules fixées après culture avec un anticorps primaire spécifique de la desmine humaine ; puis cet anticorps est reconnu par un anticorps secondaire révélé par une peroxydase colorée par du DAB. La visualisation de ce marquage se fait en lumière blanche. Cette visualisation pourrait être aussi obtenu avec des marqueurs fluorescent. Ce type de marquage présente aussi l'avantage d'être stable dans le temps et à la lumière.

Le principe de l'analyse repose sur 1) la détermination du nombre total de cellules par coloration Giemsa, 2) la détermination de la proportion de cellules marquées Desmine positive par marquage avec un anticorps primaire spécifique et 3) optionnellement la mise au point d'un système de comptage automatique afin de supprimer les variations dues à la lecture visuelle des plaques.

Dans cette perspective, un nombre équivalent de cellules est successivement déposé dans les puits de plaque 12 puits. 4 puits sont colorés au Giemsa, 4 autres puits sont testés sans anticorps primaire et 4 sont marqués avec l'anticorps spécifique de la desmine.

L'évaluation de la proportion de cellules myogéniques thérapeutiques marquées par la desmine peut être mise en oeuvre visuellement ou à l'aide d'une lecture automatisée.

Le principe de la quantification par lecture automatisée repose sur l'acquisition d'un ratio entre la surface des cellules positives pour le desmine et le nombre de cellules nucléées colorées au Giemsa.

Le bruit de fond est défini par le niveau de marquage sans anticorps primaire, il est systématiquement retranché au taux de marquage positif.

La mesure du ratio permet d'identifier et de dénombrer la proportion de cellules marquées présentant le phénotype souhaité au moment attendu.

Ce type de technologie peut être largement utilisée pour tous les types cellulaires pour lequel il existe des protéines spécifiques et des anticorps dirigés contre celle ci.

Dans une autre version de la méthode il est possible de dénombrer le nombre total de cellules en utilisant des marqueurs ubiquitaires. A titre d'exemple on peut citer, l'actine non musculaire, la vimentine, la tubuline ou des facteurs de transcription comme SP1

### Sélection par congélation et conservation par utilisation de thréhalose

La sélection des cellules et notamment des cellules musculaires, des cellules souches du muscle squelettique, des cellules souches musculaires, des cellules souches musculaires précurseurs, des myoblastes ou des cellules satellites peut également se faire, tant dans le cadre d'une extraction continue que séquentielle, par la congélation des cellules dans un milieux cryo-protectant, sélectionnant préférentiellement certaines cellules. L'ajout de tréhalose à des concentrations allant de 1 mM à 0.005M dans le milieu de congélation permet de préserver sélectivement des cellules musculaires à forte capacité de régénération. D'autres composés qui peuvent permettre une telle sélection par la congélation sont les sucres en général, la glycine, le HES (hydroxyethyl starch), le glycérol ou l'Arbutin.

De même l'ajout de thréalose ou de sucres en général, et notamment la glycine, le HES (hydroxyethyl starch), le glycérol ou l'Arbutin dans un milieu de conservation ou de preservation permet, avec ou sans congelation, la selection positive des cellules musculaires, notamment des cellules souches du muscle squelettique, des cellules souches musculaires, des cellules souches musculaires précurseurs, des myoblastes ou des cellules satellites.

### Milieux de culture améliorés

L'invention propose également des milieux de culture et notamment des milieux de sélection pour myoblastes améliorés. Il a été découvert que de manière surprenante les milieux de l'art antérieurs pouvaient être formulés pour la culture et la sélection des myoblastes et cellules musculaires souches sans utilisation d'insuline. L'invention propose donc des milieux de culture et de sélection dépourvus d'insuline. Il a été aussi découvert que de manière surprenante, l'acide ascorbique qui était utilisé par les milieux de l'art antérieurs pouvait remplacé complètement ou partiellement par de l'acide ascorbique 2-phosphate. L'acide ascorbique 2-phosphate est le précurseur de l'acide ascorbique. Cette formulation conduit à une plus grande stabilité et donc une plus grande biodisponibilité in vivo de l'acide ascorbique.

### Exemple 1 Analyse comparative des conditions d'extraction sur des prélèvements musculaires d'origine humaine sur la production de cellules à potentiel thérapeutique.

Dans cet exemple, on procède à la comparaison de deux protocoles d'extraction étant l'extraction séquentielle et l'extraction continue, objet de la présente invention.

L'extraction cellulaire est la seconde étape du processus de production cellulaire. Elle permet de libérer les cellules de l'échantillon tissulaire (biopsie) prélevé chez le patient

Les expériences ayant permis d'évaluer les deux méthodes d'extraction ont été réalisées sur 5 échantillons de tissus musculaires humains issus de patients distincts.

### Méthodes

Chaque échantillon musculaire est soumis à deux méthodes d'extraction parallèles : la méthode séquentielle (c'est-à-dire selon l'art antérieur) et la méthode continue selon l'invention. Cette étape d'extraction est suivie par des étapes d'amplification et de congélation. Les résultats de caractérisation cellulaire obtenus sont présentés ci-dessous.

### Description des deux méthodes d'extractions

La première étape est une étape commune aux deux procédés d'extraction. Une fois sortie de son milieu de transport la biopsie est placée dans une boîte de culture stérile avec quelques gouttes de DMEM/F12 afin qu'elle ne dessèche pas. Les tissus adipeux et les aponévroses sont éliminés à l'aide d'un scalpel à usage unique, puis la biopsie est découpée en petits morceaux avec des ciseaux chirurgicaux. Il s'agit de la première étape de dissociation mécanique commune au deux procédés.

### Méthode d'extraction séquentielle :

Un volume de 4 mL de Collagénase NB6 à 0.5mg/mj (solution enzymatique I) est ajouté sur les morceaux de tissus émincés. La digestion se fait à 37°C pendant 10 minutes sous une légère agitation.

La suspension est ensuite centrifugée jusqu'à 500 tr/min, et les cellules contenues dans le surnageant sont récupérées dans un tube de 50 mL. Un volume de 4 mL de DMEM/F12 + 20% sérum de veau foetal est ajouté sur les cellules.

Un volume de 4 mL de trypsine/EDTA (solution enzymatique) est ajouté sur les morceaux de tissus. La digestion se fait à 37°C pendant 10 minutes.

La suspension est ensuite centrifugée légèrement jusqu'à 500 tr/min, et les cellules contenues dans le surnageant sont collectées dans le tube de 50 mL contenant la suspension cellulaire. Un volume de 4 mL de DMEM/F12 + 20% sérum de veau foetal est ajouté dans la suspension.

L'étape de digestion à la collagénase est réalisée plusieurs fois, ainsi que l'étape de digestion à la trypsine (environ 3 fois pour chaque étape).

Après chaque étape de digestion, une goutte de suspension est déposée sur une lame pour observer la libération des sarcomères au microscope. La suspension cellulaire est centrifugée 5 minutes à 1000 tr/min. Le surnageant est éliminé et le culot est repris dans du DMEM/F12. A cette étape le dénombrement cellulaire est difficile. La normalisation se fait par le poids de tissu.

La digestion séquentielle comprend donc les étapes suivantes:
1. Tissu + solution enzymatique I.
2. Centrifugation lente pour séparer les tissus non digérés (culot) et les cellules libérées par action de la solution I (surnageant).
3. Addition d'un volume de milieu d'arrêt à un volume de surnageant.
4. tissu non digéré + solution enzymatique II (Trypsine/EDTA).
5. centrifugation lente pour séparer les tissus non digérés (culot) et les cellules libérées par action de la solution II (surnageant).
6. Addition d'un volume de milieu d'arrêt à un volume de surnageant.
7. Le tissu non digéré est prêt à être soumis à un nouveau cycle de digestion.

Ce type de digestion nécessite deux enzymes (Collagènase et Trypsine), un chélateur des ions divalents (EDTA) et un temps important de manipulation dont de nombreuses étapes de distribution et de centrifugation cellulaires rendant cette étape délicate à effectuer dans des conditions GMP. Il faut ajouter l'activité d'enzyme comme la trypsine est sensible à la présence de protéines sériques qui peuvent inhiber son activité. Cette étape est soumise à des variations et est donc dépendante de l'opérateur.

Les compositions des solutions enzymatiques sont les suivantes:

### Solution enzymatique I

*Blendzyme3 (collagénase) 25 µg* /*m, ,*/ *gentamycine, 50* µ*g*/*ml dans DMEM*/*F12.*

### Solution enzymatique II

*Gentamycine 50 µg*/*ml dans DMEM*/*F12, Trypsine 0,5 g*/*l, EDTA 0,2 g*/*l*

### Méthode d 'extraction continue

Les principes de cette méthode d'extraction continue sont l'extraction dans des conditions permettant la viabilité et la sélection cellulaire La sélection se faisant sur la capacité à survivre dans des conditions de milieux définis et sur l'adhésion préférentielle au substrat.

Cette méthode combine dans la même étape l'extraction et la sélection au moins partiellement. Dans cet exemple, la sélection se fait sur deux propriétés: la capacité à répondre à des facteurs de croissance spécifique et la capacité d'adhésion sélective au plastique de culture.

Après avoir subi, l'extraction mécanique commune aux deux méthodes, de 10 à 100 mg de tissu sont mis dans 6 mL de milieu de sélection (DMEM/F12 + αMEM + 10 % FCS + Dexaméthasone + Sodium Sélénite + Acide Ascorbique + Acide Ascorbique-2-Phosphate) en présence de collagenase (0,5 mg/mL) (dans un flacon de 25 cm2 et dans 6 ml de milieu. Cette étape peut être effectuée dans des milieux sans protéine d'origine non humaine. Le flacon de culture est placé dans un incubateur de culture (37° et 5% de CO²). Après 24 heures de digestion, le surnageant est récupéré qui contient les cellules non adhérentes et centrifugé 5 minutes à 1000 tr/min. Le culot est repris dans le milieu d'expansion suivant (DMEM/F12 + αMEM + 10 % FCS + FGF + Dexamethasone + Acide Ascorbique) et ensemencé dans des flacons de 75 cm2 pour la phase d'amplification. A cette étape nous séparons deux types de cellules: les cellules adhérentes qui sont capables d'adhérer au plastique de culture pendant les 24h de traitement enzymatique et les secondes les cellules non adhérentes qui dans ces conditions restent en suspension. Ce sont ces dernières cellules qui sont utilisées dans la suite de l'essai. Les deux types de cellules sont la source de cellules pour des amplifications ultérieures.

Les cellules adhérentes du flacon de 25 cm2 sont cultivées dans un milieu de croissance, puis amplifiées.

Le milieu combiné de digestion et de sélection utilisé est décrit dans le tableau ci-dessous.

**Tableau 1 : milieu de digestion et de sélection combiné**

| DMEM/F12 : alphaMEM (1/1) | Concentration finale |
|---|---|
| Collagénase NB6 | 0.5 mg/ml |
| Gentamycine | 50 µg/ml |
| Sérum de veau foetal | 10 % |
| Dexaméthasone | 5.10⁻⁹M |
| Acide ascorbique | 0.252 mM |
| Acide ascorbique-2-phosphate | 1 mM |
| Sodium sélénite | 250 nM |

Pour la suite de l'essai comparatif, les cellules extraites par la méthode séquentielle et la méthode continue sont soumises aux mêmes conditions expérimentales.

### Caractérisation des cellules obtenues par les deux procédés d'extraction

Une étude comparative des résultats des deux procédés d'extraction a été entreprise sur les cellules issues de cinq patients

Pour chaque patient, nous avons analysé les paramètres suivants :
- Le nombre de cellules produites par mg de tissu par jour après la première amplification.
- Le nombre de jour de culture nécessaire pour obtenir 200 millions de cellules à partir de 500mg de tissu.
- Le temps de doublement cellulaire au cours du processus d'amplification.

Les cellules extraites par la méthode séquentielle et la méthode continue sont soumises aux mêmes conditions expérimentales après leur extraction. Ces cellules sont amplifiées pendant une période variant entre 10 et 15 Jours et puis congelées. La période d'amplification permet d'étudier les conséquences quantitatives des différents procédés d'extraction sur la production cellulaire

Amplification cellulaire: l'objectif de cette étape est d'obtenir une amplification des cellules musculaires en limitant au minimum le temps de culture. Cette phase de production a lieu en système ouvert.

La culture cellulaire s'effectue en 2 phases incluant 3 étapes successives dans des plateaux ventilés de 636 cm² (Cell Stack, Corning^{®}) ou des flacons TPP^{®} de 75 cm² = 1 étape de sélection et 2 étapes d'expansions cellulaires.

La première phase d'amplification (flacons TPP^{®} de 75 cm2 ou Cell Stack, Corning^{®}) comprend la sélection et la première expansion qui dure de 8 à 9 jours la seconde phase d'amplification (flacons TPP^{®} ou plateaux Cell Stack^{®}) comprend la seconde expansion qui dure de 3 à 10 jours.

Lors de l'ensemencement initial, les cellules extraites sont cultivées en présence du milieu de sélection. La quantité cellulaire est exprimée en mg de biopsie dont les cellules sont extraites. Ce type de quantification a été retenu car la numération des cellules issues de la digestion enzymatique n'est pas possible compte tenu de la présence de nombreux débris tissulaires et de l'absence d'individualisation cellulaire. Ainsi pour 1 gramme de biopsie, l'équivalent de 200 mg est ensemencé dans 4 flacons de 75 cm² avec 20 mL de milieu de sélection dans chaque flacon et l'équivalent de 400 mg est ensemencé dans 2 plateaux 636 cm² avec 150 mL de milieu de sélection (soit 0,7 mg/cm²).

### Première expansion

Le changement de milieu a lieu au bout de 2-6 jours. Un changement de milieu a lieu 2 fois par semaine.

### Deuxième expansion

L'ensemble des cellules est détaché par action du milieu de récolte.

Le milieu de récolte est en contact avec les cellules pendant 5 à 10 minutes, puis la suspension cellulaire est reprise dans le milieu d'arrêt. Le décollement des cellules est vérifié au microscope inversé.

Après numération, les cellules sont réensemencées dans le milieu d'expansion à raison de 5x10⁴ cellules par flacon ou 4,5x10⁵ cellules par plateau (soit 700 cellules par cm²). Il s'agit de la deuxième phase d'amplification qui dure 3 -10 jours. Parallèlement à l'ensemencement des plateaux ou flacons de 75 cm², 3 flacons de 25 cm² sont également ensemencés à une densité cellulaire équivalente (17,5x10³ cellules).

A l'issue de cette deuxième phase, les cellules sont détachées par action du milieu de récolte puis diluées dans le milieu d'arrêt auquel est ajoutée de l'albumine humaine 1,6%. La nouvelle suspension cellulaire est alors lavée par centrifugation (200 g pendant 5 minutes) et reprise dans le milieu d'arrêt complété avec de l'albumine 1,6%. On procède à 2 lavages consécutifs. La numération et la viabilité cellulaire sont mesurées entre les 2 lavages.

Le nombre de flacons de la première phase est adapté en fonction du poids de la biopsie obtenu et le nombre de flacons nécessaires à la deuxième phase dépend de l'efficacité de la première expansion.

### Milieux de culture utilisés

Le milieu de culture le plus adapté pour la croissance des cellules progénitrices est un mélange volume à volume de Dulbecco Modified Eagle Medium/ Ham F 12 (DMEM/F12) et alpha Modified Eagle Medium (αMEM).

### Milieu de sélection

Il est composé de DMEM/F12 et αMEM dans une proportion 1/1 (V/V) contenant du sérum de veau foetal 10% et de la gentamycine, complété par les produits suivant à des concentrations exprimés en concentrations finales:
✔ Dexamethasone : 5.10⁻⁹ M
✔ Acide Ascorbique-2-phosphate : 1 mM
✔ Acide Ascorbique : 0.252 mM
✔ Sodium Selenite : 250 nM

### Milieu d'expansion

Il est composé de DMEM/F12 et αMEM (VN) contenant du sérum de veau foetal 10% et de la gentamycine, complété par les produits suivants en des quantités exprimées en concentration finale:
✔ FGF b : 10ng/mL
✔ Dexamethasone : 5.10⁻⁹ M
✔ Acide Ascorbique-2-phosphate: 1 mM
✔ Acide Ascorbique : 0.252 mM

### Milieu de récolte

Trypsine 0,5g/L et EDTA 0,2g/L.

### Milieu d'arrêt

DMEM/F12 seul.

### Congélation cellulaire

La congélation permet de sécuriser le procédé et de simplifier la logistique. Après la dernière centrifugation, les cellules sont suspendues de nouveau dans le milieu d'arrêt + albumine 1,6% qui sera complété par le DMSO afin d'obtenir un milieu de congélation défini, à température constante (supérieure ou égale à 20°C et inférieure à 25°C). Les cellules sont stockées dans des ampoules de congélation et les ampoules sont placées dans l'azote gazeux. Le matériel cellulaire issu du même patient est organisé en lot d'ampoules. Ce lot est composé au minimum de 3 ampoules de congélation:

### Résultats

Afin d'étudier les effets des deux procédés d'extraction (séquentielle et continue) sur le processus de production de cellules à potentiel thérapeutique, nous avons analysé 3 paramètres caractérisant les cellules produites

Pour chaque patient, nous avons analysé les paramètres suivants :
√ Le nombre de cellules produites par mg de tissu par jour après la première amplification.
√ Le nombre de jour de culture nécessaire pour obtenir 200 millions de cellules à partir de 500mg de tissu.
√ Le temps de doublement cellulaire au cours du processus d'amplification.

Les résultats obtenus sur cinq prélèvements biopsiques (N=5) indépendants par 3 expérimentateurs différents sont présentés dans le tableau suivant :

**Tableau 2 : comparaison de la croissance cellulaire en fonction de la methode d'extraction**

| Méthode d'extraction | séquentielle | continue | T test |
|---|---|---|---|
| Nombre de cellules/mg de tissu/jour | **1088+/-1351** | **4351+/-2000** | **p=0,0176** |
| Nombre de jour pour obtenir 200 millions de cellules avec 500mg de tissu | **17,5+/-1,9** | **11+/-1** | **p=0,0002** |
| Temps de doublement en h | **20+/-2.7** | **20+/-4** | **p=0,8609** |

De ces résultats, nous pouvons conclure que l'extraction continue :
• est un procédé d'extraction plus efficace et soumis à des variations interindividuelles moins importantes;
• qu'il réduit considérablement le temps de culture pour obtenir le nombre de cellules nécessaire pour la conduite de notre essai clinique;
• qu'il ne modifie pas significativement un paramètre biologique : le temps de doublement cellulaire.

L'extraction continue est un procédé plus simple à mettre en oeuvre et qu'il réduit considérablement le nombre de manipulations humaines.

### Croissance à long terme des cellules extraites selon les deux procédées d'extraction.

L'objectif est déterminer les conséquences des deux méthodes d'extraction sur la croissance à long terme et sur les capacités de sénescence des cellules ainsi produites.

Les cellules de deux patients, traitées par les deux méthodes d'extraction, ont été cultivées pendant 2 mois et ont subi 12 séries de passages cellulaires. A chaque passage le nombre de divisions cumulées a été calculé (voir figure 1).

Les résultats obtenus indiquent qu'il n'y a pas de différence pour la croissance cellulaire entre les cellules traitées par les deux méthodes d'extraction. Dans les deux conditions, on observe un nombre de divisions cumulées entre 35 et 45. Après 30 divisions, le temps de division s'allonge et les cellules extraites selon les deux procédés sénescent. En conclusion, l'extraction de type continue ne modifie pas la croissance des cellules à long terme et leur capacité de sénescence. Cette dernière propriété biologique qui indique le caractère non transformé des cellules ainsi produites est aussi un gage de sécurité du produit cellulaire thérapeutique

Ce procédé d'extraction continue permet
○ D'effectuer la digestion enzymatique dans des conditions permettant de maintenir la viabilité et les propriétés biologiques des cellules.
○ De combiner en une seule étape l'extraction et la sélection cellulaire.
○ De diminuer le nombre de manipulations humaines.
○ De diminuer 1a variabilité du procédé de production.
○ De diminuer les temps de culture pour la production du nombre de cellules nécessaires.
○ De produire des cellules ayant des capacités de croissance a long terme en maintenant leur capacités de sénescence gage de sécurité thérapeutique.

### Exemple 2 Analyse des conséquences de l'extraction continue sur les spécifications cellulaires requises pour obtenir des cellules à potentiel thérapeutique pour la conduite d'essais thérapeutiques pour la réparation de petits muscles comme le sphincter urethrale ou anal

Après le transport, le tissu musculaire est émincé mécaniquement. L'extraction cellulaire a ensuite lieu par digestion enzymatique.

L'extraction est de type continu afin d'assurer un temps de contact optimal entre les cellules et l'enzyme et dans des conditions permettant le maintien des propriétés biologiques des cellules. L'inhibition de l'action de l'enzyme se fait par lavage : dilution dans le milieu de sélection et centrifugation.

### Méthode :

Une fois transporté, le tissu musculaire est réceptionné, pesé puis émincé mécaniquement à l'aide de ciseaux chirurgicaux et pinces stérilisés à usage unique

La biopsie est placée dans une boîte avec quelques gouttes de DMEM/F12 afin qu'elle ne se dessèche pas. Le tissu adipeux et les aponévroses sont excisés à l'aide d'un scalpel à usage unique, puis la biopsie est découpée en petits morceaux avec des ciseaux chirurgicaux.

### Etape 1 Extraction/ Sélection continue:

Le tissu émincé est -mis dans le milieu de digestion enzymatique contenant de la collagenase NB6 à 0,5 mg/ml.

Puis, les fragments tissulaires sont déposés dans un plateau 636 cm² à raison de 0,4 g à 1,2 g par plateau contenant 150 ml de milieu de digestion (soit de 0,6 à 1,8 mg/cm²). Cette étape peut être effectuée sur toute une série de supports dont les flacons ventilés de 75 cm^{2.}

Le tissu musculaire est alors soumis à un cycle unique de digestion enzymatique par la collagénase NB6.

La durée de traitement enzymatique est de 24 heures. La température de traitement est de 37°C et la concentration de C0₂ à 5% . Après digestion, le surnageant contenant le milieu et cellules libérées dans le milieu est récupéré et soumis à une centrifugation (200 g pendant 5 minute). Le culot de sédimentation est repris dans du milieu de sélection, les sédiments sont réensemencés dans des supports de culture à raison de 0,7 mg/cm².

Les milieux de digestion et sélection combinés sont décrits dans le tableau ci-dessous :

**Tableau 3 : milieu de digestion/sélection combiné**

| DMEM/F12 : alphaMEM (1/1) | |
|---|---|
| Collagénase NB6 | 0.5 mg/ml |
| Gentamycine | 50 µg/ml |
| Sérum de veau foetal | 10 % |
| Dexaméthasone | 5.10⁻⁹M |
| Acide ascorbique | 0.252 mM |
| Acide ascorbique-2-phosphate | 1 mM |
| Sodium sélénite | 250 nM |

### Etape de Congélation cellulaire

L'étape de congélation optionnelle permet de sécuriser le procédé et de simplifier la logistique. Après la dernière centrifugation, les cellules sont suspendues de nouveau dans le milieu d'arrêt + albumine 1,6% qui sera complété par le DMSO afin d'obtenir un milieu de congélation défini, à température constante (supérieure ou égale à 20°C et inférieure à 25°C). Les cellules sont stockées dans des ampoules de congélation et les ampoules sont placées dans l'azote gazeux. Le matériel cellulaire issu du même patient est organisé en lot d'ampoules. Ce lot est composé au minimum de 3 ampoules de congélation.

Tout le procédé de congélation est effectué par des systèmes automatiques qui permettent de contrôler la descente de température avec une vitesse comprise entre 1°C et 2°C par minute.

Dans les 15 minutes qui suivent la préparation des cellules, les ampoules sont disposées dans la cuve de l'appareil à congélation automatique.

La congélation automatique consiste en l'utilisation d'un programmateur de descente en température appelé « DIGITCOOL ». Ce programmateur permet une descente en température progressive et automatisée. La descente en température validée lors de l'essai pilote est celle utilisée pour la congélation des cellules souches hématopoïétiques.

Le produit est introduit lorsque la cuve de l'appareil est à +10°C, puis la température de la cuve descend jusqu'au palier intermédiaire qui se situe à -40°C. Un réchauffement sensible permet la reprise de température à -25°C et la température descend de nouveau jusqu'à -120°C. Les ampoules seront alors transférées dans la vapeur d'azote. La descente en température dure 1 heure.

Les études de stabilité décrites démontrent que les cellules ayant subi une étape de cryoconservation ont des propriétés biologiques au moins aussi bonnes que les cellules n'ayant pas subies de cryoconservation. Cette bioéquivalence a été montrée par 2 études différentes chez l'animal. Le critère majeur retenu *in vitro* est l'efficacité clonale et celui évalué *in vivo* est la capacité des cellules à coloniser l'urètre de la rate.

Le milieu de congélation utilisé est composé de :
DMEM/F12 en un rapport 1/1 (v/v).
Facteur cryoprotecteur : DMSO 5%.
Albumine humaine 1,6% (16 g/L).

### Contrôles

L'ensemble des contrôles est décrit dans le tableau ci-dessous :

**Tableau 4 : contrôle de qualité**

| ***Etape de production*** | ***Contrôle qualité*** |
|---|---|
| Eminçage du fragment biopsique | STATUT VIROLOGIQUE ET PESEE |
| Extraction cellulaire par digestion enzymatique | OBSERVATION MICROSCOPIQUE CONTROLE MICROBIOLOGIQUE |
| Décollement des cellules | NUMERATION |
| Deuxième amplification cellulaire | CONTROLE « IN PROCESS » NUMERATION-VIABILITE CONTROLE MICROBIOLOGIQUE |
| Congélation | CARACTERISATION du lot. |

### Contrôle sur la première suspension cellulaire amplifiée (Numération)

A l'issue de la première phase d'amplification, les cellules sont récoltées et contrôlées par une numération cellulaire. La numération sur un échantillon est effectuée par la lecture sur une lame Malassez du nombre de cellules obtenues.

Ce contrôle conditionne l'étape d'ensemencement cellulaire pour la seconde expansion.

### Contrôle sur la deuxième suspension cellulaire (Numération)

Ce contrôle appelé contrôle « *in process*» est effectué en cours de seconde amplification (3^{ème} ou 4^{ème} jour et 6 ou 7^{ème} jour) sur un flacon de 25 cm² destiné à cet usage.

Cette étape clef est critique pour déterminer le jour de la congélation du lot cellulaire. La densité ciblée est de 50 000 à 150 000 cellules par cm², elle pourra être obtenu entre 3 à 10 jours.

Les milieux de digestion/sélection combinés sont décrits ci-dessous.

**Tableau 5 : composition des milieux de digestion/sélection combiné incluant la solution enzymatique I**

| **Nom du composant** | **Fournisseur** | **Origine** | **Fonction** | **Numéro d'enregistrement** | **Référence aux standards** | **Unité / quantité ou pourcentage ou concentration finale** |
|---|---|---|---|---|---|---|
| DMEM/F12 | Cambrex | NOA | Milieu de culture | BE12-719F | | ½ Volume |
| alphaMEM | Cambrex | NOA | Milieu de culture | BE12-169F | | ½ volume |
| Gentamycine | Schering Plough | | antibiotique | AMM 322 349 1 | Pharmacopée Européenne édition en cours | 50 mg/L |
| Collagenase (NB6) | Serva | OA | Digestion enzymatique I | | | 500 mg/L |
| Sérum de veau foetal | Cambrex | OA + irradié | Facteurs de croissance | US14-471F | | 10% |
| Acide Ascorbique | Roche | chimique | antioxydant | AMM 342135-7 ou AMM 557160-6 | Pharmacopée Européenne édition en cours | 0,252 mM |
| Acide Ascorbique-2-Phosphate | Cambrex | chimique | Antioxydant | A8960 | | 1mM |
| Dexamethasone | Merck | chimique | cofacteur de croissance | AMM 550977-7 Ou AMM 309740-2 | Pharmacopée Européenne édition en cours | 5 10⁻⁹ M |
| Sodium Selenite | Sigma | NOA | cofacteur | S5261 | | 250 nM |

La composition du milieu de congélation est décrite ci-dessous.

**Tableau 6: composition du milieu de congélation. Le volume de DMSO ajouté correspond à 5% du volume du produit final.**

| **Nom du composant** | **Fournisseur** | **Origine** | **Fonction** | **Numéro d'enregistrement** | **Référence aux standards** | **Unité / quantité ou pourcentage dans le produit final** |
|---|---|---|---|---|---|---|
| Albumine 20% | LFB | humaine | Maintien de la pression oncotique | AMM 558 451-4 | Pharmacopée Européenne Version actuelle | 16 g/L |
| DMSO | Braun | chimique | Cryoprotecteur | 9575 H | Pharmacopée Européenne Version actuelle | 5% |
| DMEM/F12 | Cambrex | chimique | Milieu cellulaire | BE12-719F | Pharmacopée Européenne Version actuelle | qsp 100% |

### Résultats

L'extraction continue permettant d'obtenir des cellules potentiellement thérapeutiques dans de meilleures conditions de sécurité, nous avons analysé les spécifications des cellules produites avec cette technique sur des prélèvements de tissu musculaire issus de trois patients.

Les résultats sont indiqués dans le tableau suivant:

**Tableau 7 : propriétés des cellules extraites par l'extraction continue. Nd = non déterminé**

| Paramètres | Résultats attendus | Echantillon 1 | Echantillon 2 | Echantillon 3 |
|---|---|---|---|---|
| Viabilité | >70% | nd | 97% | 74% |
| Taux de recouvreme nt | > 50% | 98% | nd | 79,9 |
| Efficacité clonale | > 10% | 64% | 32% | 35% |
| Temps de doublement (h) | Entre 13.5 et 30 | 19,3 | 17 | 16 |
| Expression de la desmine | >50 | 153 | 118 | 230 |

Pour tous les paramètres analysés, les cellules produites avec la technique d'extraction répondent aux spécifications exigées pour la conduite de notre essai de thérapie cellulaire.

En conclusion, l'extraction continue permet une production de cellules potentiellement thérapeutiques dans des conditions de sécurité améliorées et ayant les spécifications cellulaires requises pour la conduite d'essais thérapeutiques pour la réparation tissulaire de muscle par thérapie cellulaire

### Exemple 3: Rôle du temps de digestion/ sélection sur l'efficacité de l'extraction cellulaire pour la production cellulaire

Dans cet exemple, nous avons analysé le rôle du temps de digestion/sélection enzymatique sur le nombre de cellules extraites capables de proliférer.

### Méthode

La méthode d'extraction est décrite dans l'exemple 2. Le paramètre étudié est le nombre de cellules extraites par mg de tissu par jour après une première amplification.

### Résultats

Nous avons choisi de prendre comme référence, le nombre de cellules obtenues après 24h qui représente le 100%. Les données obtenues sur deux patients sont présentées dans le tableau qui suit.

**Tableau 8 : nombre de cellules en fonction du temps après une première amplification**

| Temps de Digestion/sélection combiné | Nombre de cellules/ mg de tissu/ Jours |
|---|---|
| 6h | 69% |
| 24h | 100% |
| 30h | 407% |

Le nombre de cellules ainsi obtenues est dépendant du temps de digestion/sélection. Le passage de 24h à 30h permet d'augmenter l'efficacité de l'extraction par un facteur 4. Le temps moyen de divisions des cellules étant de l'ordre de 24h, il est possible de réduire de deux jours le temps de culture pour produire le nombre nécessaire de cellules pour un essai thérapeutique. Le temps d'extraction sélection est une étape importante du procédé et son efficacité est dépendante du temps de digestion sélection.

### Exemple 4 Sélection cellulaire par l'adhésion différentielle.

Dans cet exemple nous allons analyser les cellules sélectionnées et les cellules non sélectionnées par le procédé d'extraction dite continue.

### Méthodes

Les prélèvements ont pour origine des déchets opératoires obtenus au décours d'opérations chirurgicales. Les prélèvements sont obtenus de manière stérile et sont transportés dans un milieu permettant le maintien de la viabilité tissulaire. Une fois arrivés au laboratoire, les tissus sont soumis aux différentes opérations. Les tissus cellulaires sont toujours composés de nombreux types cellulaires, un des objectifs de ce procédé est d'augmenter l'homogénéité des cellules produites. Pour réaliser ces objectifs, il existe de nombreuses stratégies. La sélection cellulaire peut être effectuée sur des propriétés biologiques (capacités de survie, cytoxicité sélective, expression génétique, capacité d'adhésion) sur des propriétés moléculaires (présence de marqueurs membranaires). Les techniques de sélections peuvent être soit des techniques de culture soit des techniques faisant appel au tri magnétique ou à la cytométrie de flux.

Pour cette étude, nous avons utilisé les propriétés d'adhésion différentielle au plastique traité pour la culture cellulaire. Les étapes de digestion et de sélection ont été effectuées selon l'exemple 1.

Pendant l'étape de digestion/sélection combinée enzymatique, les fragments tissulaires sont placés dans des boites de plastique traitée pour la culture cellulaire. Par ce procédé il est donc possible de séparer les cellules qui vont adhérer au substrat (cellules adhérentes) et les cellules qui ne vont pas adhérer au substrat (cellules non adhérentes). Après 24 h de culture les cellules non adhérentes ont été séparées des cellules adhérentes.

Les cellules non adhérentes sont récupérées par pipetage. A cette étape le milieu contient les facteurs de croissance et les cellules libérées par la digestion enzymatique. Cette suspension cellulaire de cellules non adhérentes ainsi obtenue est une source de cellules pour la conservation cellulaire, la sélection et l'amplification cellulaire comme pour l'exemple 1

Les cellules adhérentes sont les cellules qui restent attachées aux substrats après pipetage. Les cellules sont alors soient amplifiées directement soit repiquées à l'aide solution de trypsine EDTA. Ces cellules sont une source de cellules pour la conservation cellulaire, la sélection et l'amplification cellulaire. Les conditions de croissance sont semblables à celles utilisées pour les cellules adhérentes

### Résultats

Les cellules non adhérentes et les cellules adhérentes sont mises en culture. Leurs caractéristiques sont indiquées sur le tableau suivant. Les techniques utilisées sont décrites dans l'exemple 1.

**Tableau 9 : caractéristique des cellules selon leur adhérence.**

| | Cellules non adhérentes | Cellules adhérentes |
|---|---|---|
| **Nombre de cellules/mg de tissu/jour après la 1^{ère} amplification** | 3 222 | 596 |
| Temps de doublement en heure | 20,7 | 28,2 |
| Expression de la desmine (c) | élevé | Non détectable |

Le procédé de sélection utilisant l'adhésion préférentielle au plastique traité pour la culture cellulaire permet de séparer deux populations cellulaires. Les deux populations présentent des capacités de croissance en culture. Les cellules non adhérentes sont 5 fois moins nombreuses que les cellules adhérentes et leur temps de doublement est inférieur de près de 30%. D'autre part, elles sont très différentes pour l'expression de la desmine. Les cellules non adhérentes expriment dans leur quasi-totalité la desmine, les cellules adhérentes présentent l'image inverse. Pour résumer ce procédé de sélection permet de séparer les cellules musculaires (cellules non adhérentes) des cellules non musculaires (cellules adhérentes), issues d'une biopsie musculaire. Ce procédé est simple à mettre en oeuvre et peut être réalisé dans des conditions GMP, conditions nécessaires pour la production de cellules thérapeutiques

### Exemple 5 Digestion/ Sélection cellulaire sur fragments tissulaires congelés,

La possibilité de congeler les fragments tissulaires permet des simplifications logistiques des procédés de production cellulaire.

Les prélèvements ont pour origine des déchets opératoires obtenus au décours d'opérations chirurgicales. Les prélèvements sont obtenus de manière stérile et sont transportés dans un milieu permettant le maintien de la viabilité tissulaire. Une fois arrivés au laboratoire, les tissus sont soumis aux différentes opérations. Les fragments tissulaires ont été directement congelés après l'étape de dissociation mécanique.. La congélation peut être pratiquée en présence de composés sérique ou de milieux définis. Les échantillons ainsi obtenus sont conservés dans des vapeurs d'azote liquide.

La congélation permet de sécuriser le procédé et de simplifier la logistique. Après la dernière centrifugation, les cellules sont suspendues de nouveau dans le milieu d'arrêt + albumine 1,6% qui sera complété par le DMSO afin d'obtenir un milieu de congélation défini Sans protéines d'origine animale, à température constante (supérieure ou égale à 20°C et inférieure à 25°C). Les cellules sont stockées dans des ampoules de congélation et les ampoules sont placées dans l'azote gazeux. Le matériel cellulaire issu du même patient est organisé en lot d'ampoules La décongélation est rapide et s'effectue entre 35°C et 37°C.

### Méthodes

Une fois sortie du container à azote les fragments tissulaires sont rapidement décongelés à la température de 37°.

Ces fragments sont soumis à une digestion / sélection de type continu comme dans l'exemple 1 et 2.

### Résultats

Le tableau ci-dessous compare les résultats obtenus avec des fragments fais ou congelés issu d'un prélèvement identique de tissu humain Les fragments ont été congelés dans différents milieux de congélation. La concentration de DMSO est identique (5% du volume final) dans toutes les conditions. Certaines de ces conditions sont exemptes de toutes protéines d'origine animale.

**Tableau 10 : pourcentage de cellules préservées en fonction de milieux de congélation.**

| | Nombre de cellules obtenues / Jours/mg de tissu | Pourcentage |
|---|---|---|
| Tissu frais sans étapes de congélation préalable | 3322 cellules/mg/jour | 100% |
| DMEM/F12 + albumine + 5% DMSO | 2240 cellules /mg/jour | 67% |
| DMEM/F12: 95% FCS+ 5% DMSO | 1 440 cellules /mg/jour | 43% |

Il est important de constater que dans un milieu totalement exempt de protéines d'origine animale, il est possible de préserver 67% des cellules capables d'être amplifiées et donc de fournir des cellules thérapeutiques. La présence de sérum de veau dans le milieu de conservation diminue l'efficacité du milieu de congélation.

Dans la deuxième partie de l'étude nous nous sommes intéressés aux critères qualitatifs et quantitatifs des cellules ainsi obtenues après congélation du tissu. Dans tous les cas il est possible d'observer des cellules desmine positives.

**Tableau 11 comparaison de l'effet de la congélation des tissus**

| **Condition** | DMEM/F12 + albumine + 5% DMSO | DMEM/F12: 95 % FCS+ 5% DMSO |
|---|---|---|
| **Quantité de tissus congelés** | 100 | 100 |
| **Nombre de jours de 1^{ère} amplification** | 10 | 10 |
| **Nombre de cellules après la 1^{ère} amplification** | 2 240 000 | 1 440 000 |
| **Nombre de cellules/mg de tissu/jour après la 1^{ère} amplification** | 2240 | 1 440 |
| **Nombre de cellules ensemencées pour la 2^{ème} amplification** | 200000 | 200000 |
| **Nombre de jours de 2^{ème} amplification** | 7 | 7 |
| **Nombre de cellules produites après la 2^{ème} amplification** | 57 040 000 | 49 040 000 |
| **Temps de doublement (heures)** | 20,6 | 21,2 |
| **Nombre de cellules/cm2 après la 2^{ème} amplification** | 190 133 | 163 467 |
| **Nombre de jours pour obtenir 200 millions de cellules avec 500 mg de tissu** | 13,6 | 14,2 |

En absence de protéines d'origine non humaine (DMEM/F12 + albumine + 5% DMSO), il est possible de conserver des tissus en sélectionnant les cellules musculaires. Dans ces conditions, il est possible d'obtenir un meilleur rendement (comparé a celui observé en présence de sérum de veau fetal) sélectif de digestion/sélection et d'accroître la capacité de sélectionner des cellules musculaires. Dans ces conditions, les cellules sont capables d'autorenouvellement et d'exprimer la desmine et de former des précurseurs des fibres musculaires les myotubes. Dans ces conditions en absence de protéines d'origine animale, il suffirait de 16 jours pour accumuler 200 millions de cellules potentiellement thérapeutiques

La congélation direct du tissu musculaire en absence de protéines d'origine permet de conserver des cellules ayant un potentiel thérapeutiques dans des conditions compatible avec les bonnes pratiques de laboratoire nécessaires pour la production de cellules thérapeutiques.

### Exemple 6 Conservation cellulaire par congélation après l'étape de digestion et sélection combinée.

Dans cet exemple, nous avons testé la possibilité de conserver par congélation les cellules par congélation après la première étape de digestion et sélection. La possibilité de conserver les cellules à plusieurs étapes du procédé de production est importante pour des raisons techniques réglementaire et administratives.

### Méthodes

Comme pour les exemples précédents, les prélèvements ont pour origine des déchets opératoires obtenus au décours d'opérations chirurgicales. Les prélèvements sont obtenus de manière stérile et sont transportés dans un milieu permettant le maintien de la viabilité tissulaire

La biopsie est placée dans une boîte avec quelques gouttes de DMEM/F12 afin qu'elle ne se dessèche pas. Le tissu adipeux et les aponévroses sont excisés à l'aide d'un scalpel à usage unique, puis la biopsie est découpée en petits morceaux avec des ciseaux chirurgicaux.

### Etape 1 Digestion/ Sélection continue:

Le tissu émincé est mis dans le milieu de digestion enzymatique contenant de la collagenase NB6 à 0,5 mg/ml.

Puis, les fragments tissulaires sont déposés dans un plateau 636 cm² à raison de 0,4 g à 1,2 g par plateau contenant 150 ml de milieu de digestion (soit de 0,6 à 1,8 mg/cm²). (liasser la possibilité d'utiliser des flacons de 75cm.

Le tissu musculaire est alors soumis à un cycle unique de digestion enzymatique par la collagénase NB6.

La durée de traitement enzymatique est de 24 heures. La température de traitement est de 37°C. Après digestion, le surnageant contenant le milieu + cellules libérées dans le milieu est récupéré et soumis à une centrifugation (200 g pendant 5 minute).

Les milieux de digestion/ Sélection combiné sont composé de :

| DMEM/F12 : alphaMEM (1/1) | Concentration finale |
|---|---|
| Collagénase NB6 | 0.5 mg/ml |
| Gentamycine | 50 µg/ml |
| Sérum de veau foetal | 10 % |
| Dexaméthasone | 5.10⁻⁹M |
| Acide ascorbique | 0.252 mM |
| Acide ascorbique-2-phosphate | 1 mM |
| Sodium sélénite | 250 nM |

Le surnageant cellulaire contenant les cellules non adhérentes sont après une étape de lavage sont congelées dans le milieu de croissance décrit ci dessous

### Milieu de congélation :

DMEM/F12
Facteur cryoprotecteur : DMSO 5%
Albumine humaine 1,6% (16 g/L)

### Résultats

Le tissu est soumis à une procédure de digestion et sélection combinée selon l'exemple 1. Après 24h d'extraction sélection les cellules présentes dans le surnageant sont soient cultivées directement comme dans l'exemple 1, soient congelées dans différentes conditions expérimentales.

Après décongélation, les capacités de croissance des cellules sont analysées et comparées aux résultats obtenus avec des cellules n'ayant pas subies de congélation les résultats sont montrés dans le tableau suivant :

**Tableau 12 : pourcentage de cellules préservées en fonction des milieux de congélation.**

| | Nombre de cellules obtenues/ Jours/mg de tissu | Pourcentage |
|---|---|---|
| Absence de congélation | 7291 | 100% |
| DME/F12, Albumine, DMSO 5% | 2857 | 39% |
| DME/F12, Albumine, FCS 10%, DMSO 5% | 2343 | 32% |
| DME/F12, Albumine, FCS 10%, DMSO 10% | 2514 | 34% |
| DME/F12, FCS, 95 % DMSO 5% | 4486 | 61% |

Après la digestion et sélection combinée, les cellules directement congelées peuvent de source de cellules pour la production de cellules thérapeutiques. Cependant, l'efficacité du procédé dans ces conditions est partielle et est dépendante des conditions de congélation. D'un point de vue qualitatif, la congélation ne modifie les propriétés des cellules ainsi conservées. Les cellules extraites de tissu congelé possèdent des caractéristiques identiques à celles des cellules extraites de tissu frais que cela soit pour les propriétés de croissance ou les propriétés de différenciation.

La conservation par congélation des cellules après la digestion et sélection ne modifie le potentiel des cellules. Ces cellules présentent à la fois des capacités d'auto-renouvelement et des capacités de différenciation au moins égales aux cellules extraites de tissu frais. Cette étape du procédé permet de conserver des cellules dès l'étape d'extraction sélection sans modifier le potentiel des cellules conservées Ces cellules ainsi conservées peuvent être des sources de cellules pour la thérapie cellulaire et la constitution de banque de tissu.

### Exemple 7 : Le tréhalose un agent pour la conservation sélective des cellules musculaires dans un tissu immédiatement congelé

Les méthodes utilisées pour cet exemple sont similaires aux méthodes utilisées pour l'exemple 5. Dans le tableau suivant, nous nous sommes intéressé aux aspects quantitatifs des cellules extraites de tissu directement congelé. Dans cet exemple la concentration de tréhalose est de 0.2M . Il est possible de faire varier la concentration de 1mM à 1M. Les résultats sont indiqués dans le tableau suivant :

**Tableau 13: comparaison entre le nombre de cellules obtenues après congelation avec ou sans tréhalose.**

| | Nombre de cellules obtenues / Jours/mg de tissu | Pourcentage |
|---|---|---|
| Tissu frais sans étapes de congélation préalable | 3322 cellules/mg/jour | 100% |
| DMEM/F12 + albumine + 5% DMSO | 2240 cellules/mg/jour | 67% |
| DMEM/F12 + albumine + 5% DMSO + 0.2 M Tréhalose | 600 cellules/mg/jour | 18% |

En présence de thréalose des cellules peuvent être extraites des tissus directement quoiqu'en moins grand nombre. Pour une part, cet effet est dû à la concentration importante de tréhalose (0.2M)utilisée dans cette condition expérimentale. Les résultats du tableau suivant portent sur les capacités de croissance des cellules extraites des tissus congelés en présence et en absence de tréhalose.

**Tableau 13 Comparaison de l'effet de la congélation des tissus en présence de tréhalose sur l'extraction et l'amplification cellulaire.**

| **Condition** | DMEM/F12 + albumine + 5% DMSO | DMEM/F12 + albumine + 5% DMSO + 0.2M Tréhalose |
|---|---|---|
| **Quantité de tissus congelés** | 100 | 100 |
| **Nombre de jours de 1^{ère} amplification** | 10 | 10 |
| **Nombre de cellules après la 1^{ère} amplification** | 2 240 000 | 600 000 |
| **Nombre de cellules/mg de tissu/jour après la 1^{ère} amplification** | 2240 | 600 |
| **Nombre de cellules ensemencées pour la 2^{ème} amplification** | 200 000 | 200 000 |
| **Nombre de jours de 2^{ème} amplification** | 7 | 7 |
| **Nombre de cellules produites après la 2^{ème} amplification** | 57 040 000 | 29 040 000 |
| **Temps de doublement (heures)** | 20,6 | 23,4 |
| **Nombre de cellules/cm2 après la 2^{ème} amplification** | 190 133 | 96 800 |
| **Nombre de jours pour obtenir 200 millions de cellules avec 500 mg de tissu** | 13,6 | 15,9 |

Les capacités de croissance des cellules extraites en présence de tréhalose sont très voisines des cellules extraites de tissus non congelés. En effet, il suffit de 16 jours pour accumuler 200 millions de cellules à partir de 500mg de tissu congeler en présence de tréhalose. L'absence de ce sucre réduit la période uniquement de 2 jours. La congélation en présence de tréhalose ne modifie les capacités de croissance des cellules extraites. L'efficacité clonale est le rapport entre le nombre de colonie observées et le nombre initial de cellules initiales ensemencées. Le tableau suivant montre que la congélation en présence de tréhalose ne modifie pas la capacité de croissance clonale.

**Tableau 14: comparaison de l'efficacité clonale des cellules congelées avec ou sans tréhalose.**

| | Sans Tréhalose | | | | | | Avec tréhalose | | | | | |
|---|---|---|---|---|---|---|---|---|---|---|---|---|
| nb de jours de culture après décongélation | 13 | | | 21 | | | 13 | | | 21 | | |
| Nombre de colonies | 19 | 24 | 27 | 27 | 13 | 17 | 22 | 24 | 24 | 23 | 20 | 15 |
| Efficacité clonale (%) | 23,3 | | | 19 | | | 23,3 | | | 19,3 | | |

Dans le tableau suivant, nous avons analysé la capacité des cellules extraites après congélation à exprimer la desmine marqueur spécifique des cellules musculaires. La concentration de tréhalose était de 0.2M. Après conservation en présence ou en absence de tréhalose les tissus ont été soumis à une étape de digestion et de sélection. Les cellules ont été analysées pour l'expression de la desmine. Les cellules ont été marquées par immunoperoxydase à l'aide d'un anticorps spécifique reconnaissant la desmine protéine spécifique des cellules musculaires.

Les cellules extraites et sélectionnées de tissu congelé en présence de tréhalose expriment dans leur très grande majorité la desmine et elles forment dans une plus grande proportion des myotubes cellules précurseurs de la fibre musculaire. La figure 2 indique très clairement que les cellules extraites de tissu congelé en présence de tréhalose expriment dans leur très grande majorité la desmine. Les résultats obtenus par analyse d'image présentés dans le tableau suivant le confirment.

**Tableau 15: comparaison de la quantité de cellules exprimant la desmine selon que la congélation est faite avec ou sans tréhalose.**

| Tissu congelé | Sans Tréhalose | Avec Tréhalose |
|---|---|---|
| Desmine | 522+/-102 | 1544+/-286 |

Il est très remarquable de constater qu'après congélation en présence de tréhalose, les cellules dans leur très grande majorité sont capables d'exprimer la desmine. Dans ce sens, il s'agit d'un agent qui améliore la sélection des cellules musculaires avec un potentiel thérapeutique au cours de l'étape de conservation tissulaire.

La congélation dans un milieu contenant de la tréhalose permet donc de conserver et de sélectionner des cellules de précurseurs musculaires, des myoblastes ou des cellules satellites de façon à obtenir une population enrichie et avec un potentiel de régénération cellulaire et donc de réparation .

La conservation des fragments tissulaires par congélation ne modifie les potentiels des cellules extraites.La présence de tréhalose permet de préserver sélectivement les cellules présentes dans le tissu qui vont donner des cellules capables d'autoréplication et de donner des cellules exprimant la desmine. Ces cellules présentent à la fois des capacités d'auto renouvellement et des capacités de différenciation au moins égales aux cellules extraites de tissus frais. Cette étape du procédé permet de conserver des fragments tissulaires sans modifier le potentiel des cellules présentes dans ces fragments. Ces fragments ainsi conservés peuvent être des sources de cellules pour la thérapie cellulaire et la constitution de banque de tissu.

Constituer des banques de tissus préservant la viabilité des cellules constituant les tissus est un objectif à la fois cognitif et industriel. Le procédé décrit permet de préserver l'organisation tissulaire et la viabilité cellulaire sélective des cellules musculaires.. Avec la présente invention, il est possible de construire des banques de tissu cellulaire sans étapes d'extraction enzymatique et sans étapes de culture cellulaire.

## Revendications

1. Un procédé de sélection de cellules comprenant au moins une étape au moins partiellement combinée de digestion enzymatique et de sélection en culture.

2. Un procédé selon la revendication 1 dans lequel la source des cellules est un échantillon tissulaire et de préférence une biopsie musculaire.

3. Un procédé selon la revendication 1 dans lequel la source des cellules est une culture de cellules et de préférence un agrégat de cellules en culture.

4. Un procédé selon les revendications 1 à 3 dans lequel les cellules sélectionnées sont des cellules musculaires.

5. Un procédé selon les revendications 1 à 4 dans lequel les cellules sélectionnées sont des cellules souches du muscle squelettique, des cellules souches musculaires, des cellules souches musculaires précurseurs, des myoblastes ou des cellules satellites.

6. Un procédé selon les revendications 1 à 5 dans lequel la sélection se fait au moins par la culture dans un milieu de sélection.

7. Un procédé selon les revendications 1 à 5 dans lequel la sélection se fait au moins par l'adhésion à un substrat en culture.

8. Un procédé selon les revendications 1 à 5 dans lequel la sélection se fait au moins par l'adhésion à un substrat et par culture dans un milieu de sélection.

9. Un procédé selon les revendications 7 à 8 dans lesquelles le substrat est choisi dans le groupe consistant en le verre, le plastique traité, des substrats synthétiques, des cellules nourricières, des anticorps, des peptides et les constituants de la matrice extra cellulaire, de préférence la Laminine, la Fibronectine, la Vitronectine.

10. Un procédé selon la revendication 9 dans lequel les cellules adhérentes sont sélectionnées.

11. Un procédé selon la revendication 9 dans lequel les cellules non adhérentes sont sélectionnées.

12. Un procédé selon l'une des revendications précédentes dans lesquelles la sélection se fait par culture dans un milieu de sélection comprenant au moins de la dexaméthasone, du sélénium, et un ou plusieurs composés choisi dans le groupe consistant en l'acide ascorbique-2-phosphate, l'acide ascorbique et leurs mélanges.

13. Un procédé selon l'une des revendications précédentes dans lequel le milieu de culture comprend l'enzyme utilisée pour la digestion enzymatique.

14. Un procédé selon l'une des revendications précédentes dans lequel le milieu de sélection comprend l'enzyme utilisée pour la digestion enzymatique.

15. Un procédé selon l'une des revendications précédentes dans lequel l'extraction enzymatique utilise une enzyme sélectionnée dans le groupe consistant en la collagènase, la protéase neutre, la pronase, la trypsine et leurs mélanges.

16. Un procédé selon l'une des revendications précédentes dans lequel l'étape combinée d'extraction enzymatique et de sélection dure au moins 3 heures, de préférence au moins 6 heures, de manière préférée au moins 12 heures et de manière très préféré au moins 24 heures, et de manière préférée entre toutes au moins 48 heures.

17. Un procédé de sélection de cellules musculaires par conservation en présence d'un composé choisi dans le groupe consistant en les sucres, la tréhalose, la glycine, le HES (hydroxyethyl starch), le glycerol et l'arbutin.

18. Un procédé selon la revendication 17 dans lequel la source des cellules est un échantillon tissulaire et de préférence une biopsie musculaire.

19. Un procédé selon la revendication 17 dans lequel la source des cellules est une culture de cellules et de préférence un agrégat de cellules en culture.

20. Un procédé selon les revendications 17 à 19 dans lequel les cellules sélectionnées sont des cellules souches du muscle squelettique, des cellules souches musculaires, des cellules souches musculaires précurseurs, des myoblastes ou des cellules satellites.

21. Un procédé selon les revendications 17 à 20 dans lequel la conservation comprend au moins une étape de congélation.

22. Un procédé selon la revendication précédente dans laquelle la congélation se fait en présence de tréhalose.

23. Un procédé selon l'une des revendications 21 ou 22 dans laquelle la concentration de tréhalose est comprise entre 1mM à 1M de préférence 0.2M.

24. Cellules susceptibles d'être obtenues par les procédés de sélection de cellules des revendications précédentes.

25. Cellules musculaires susceptibles d'être obtenues par les procédés de sélection de cellules des revendications précédentes.

26. Cellules musculaires susceptibles d'être obtenues par les procédés de sélection de cellules selon l'une des revendications précédentes pour leur utilisation en thérapie cellulaire.

27. Cellules musculaires selon la revendication 26 pour leur utilisation dans le traitement fonctionnel des muscles.

28. Cellules musculaires selon la revendication précédente pour leur utilisation dans le traitement fonctionnel des petits muscles.

29. Cellules musculaires selon la revendication précédente pour leur utilisation dans le traitement fonctionnel des sphincters.

30. Cellules musculaires selon la revendication précédente pour leur utilisation dans le traitement fonctionnel de l'incontinence urinaire

31. Cellules musculaires selon la revendication 29 pour leur utilisation dans le traitement fonctionnel de l'incontinence anale.

32. Un milieu de culture substantiellement dépourvu d'insuline comprenant au moins de la dexaméthasone, du sélénium, et un ou plusieurs composés choisi dans le groupe consistant en l'acide ascorbique-2-phosphate, l'acide ascorbique et leur mélanges.

33. Un milieu de culture comprenant au moins de la dexaméthasone, du sélénium, de l'acide ascorbique-2-phosphate et de l'acide ascorbique.

34. Un milieu de culture selon les revendications 32 ou 33 comprenant en outre du sérum, de préférence du sérum de veau foetal ou du sérum humain.

35. Un milieu de culture selon la revendication précédente dans laquelle la concentration de sérum en volume est au moins 5% et de manière préférée 10%.

36. Un milieu de culture selon la revendication précédente comprenant au moins de la dexaméthasone à une concentration d'environ 5.10⁻⁹ M, de l'acide ascorbique-2-phosphate à une concentration d'environ 1 mM, de l'acide ascorbique à une concentration de 0.252 mM et du sodium selenite à une concentration de 250 nM.

37. Un milieu de culture selon les revendications 32 à 36 comprenant en outre une enzyme pour la digestion enzymatique.

38. Un milieu de culture selon la revendication précédente dans lequel l'enzyme est choisie dans le groupe consistant en la collagènase, la protéase neutre, la pronase, et la trypsine.

39. Un milieu de conservation cellulaire comprenant du tréhalose et du DMSO.

40. Un milieu de conservation cellulaire selon la revendication précédente dans laquelle la concentration finale de tréhalose est comprise entre 1mM à 1M de préférence 0.2M.

41. Un milieu de conservation cellulaire selon l'une des revendications 39 à 40 comprenant en outre du sérum.

42. Un milieu de conservation cellulaire selon la revendication précédente comprenant de 0 à 90% de sérum en volume, de 0 à 20% de DMSO en volume et de 1 mM à 1M de tréhalose.

43. Un milieu de conservation cellulaire selon l'une des revendications 39 à 40 comprenant en outre de l'albumine.

44. Un milieu de conservation cellulaire selon la revendication précédente comprenant de 1 à 50% d'albumine en volume, de 0 à 20% de DMSO en volume et de 1 mM à 1 M de tréhalose.
